# EUROPEAN PATENT APPLICATION

(11) **EP 3 875 037 A1**
(43) Date of publication of application: **08.09.2021**
(21) Application number: 20162826.0
(22) Date of filing: 12.03.2020
(51) Int. Cl.: A61B 10/00, A41D 13/11, A62B 23/02

(54) **MEANS FOR COLLECTING A SAMPLE FROM THE RESPIRATORY TRACT**

(30) Priority: 02.03.2020 EP 20160506
(71) Applicant: Solvamed GmbH, 18057 Rostock (DE); Smotec GmbH, 65185 Wiesbaden (DE)
(72) Inventor: The designation of the inventor has not yet been filed
(74) Representative: Patentanwälte Bauer Vorberg Kayser

(57) **Abstract**

The present invention relates to a face mask for collecting a sample from a patient comprising
- a mask body comprising at least one outer layer able to cover at least mouth of a patient's face,
- an insert attached to said mask body at the side facing the patient's mouth and covered with a material able to accumulate pathogen particles.

## Description

The present invention relates to means for collecting samples for detection of pathogens infecting the respiratory contract, particularly a face mask for collecting samples for detection of pathogens infecting the respiratory tract.

The detection of pathogens, i.e. causing bacterial or viral infections, in particular novel and potentially more harmful viruses, is of vital importance to be able to control the spread of the respective pathogen in the community. In order to detect an infection with a virus infecting the respiratory system, such as SARS-1, MERS, influenza or the novel coronavirus SARS-CoV-2 (Covid19), adenovirus, picornaviridae, paramyxoviridae or bacterial pathogens such as tuberculosis, pneumococcus, haemophilus influenzae, Moraxella catarrhalis or pertussis, normally a sample is taken from a patient by having said patient cough and taking mucus with a cotton swab or just by swiping inside the oropharyngeal regions. Said swab is then put into a tube prior to detecting the virus using PCR. In particular the detection of SARS-CoV-2 has been difficult. Often patients detected as negative, later on turned out to be infected. In many cases there is only a low amount of virus yield using the cotton swap method. In SARS 1 the method achieved only a sensitivity of 60%. Furthermore, using the traditional method involves a high danger for the doctor or nurse to get an infection during the process, which may provoke a sneezing or cough by the patient.
Accordingly, there is a need for a more enriched sample to reduce false negative results while improving safety of medical personnel.

This technical problem is at least in part solved by the present invention.

Accordingly, in one embodiment, the present invention relates to a face mask for collecting a sample from a patient comprising a mask body comprising at least one outer layer able to cover at least the mouth of a patient's face, and an insert attached to said mask body at the side facing the patient's mouth and covered with a material able to immobilize pathogen particles, e.g. bacteria or viruses, or cells such as cancer cells.

A sample to be collected may comprise particles or droplets carried with a patient's breath as well as mucus, saliva, blood or a mixture of any of the foregoing, or any other liquid or solid material transported through the respiratory pathways, in particular mouth, but also nose. Such material may contain virus particles which will be directed to the insert of the face mask and immobilized on the coating material of said insert.

Without wishing to be bound to a scientific theory, Applicant believes that there is a number of drawbacks in current means for collecting samples. Some viruses infecting the respiratory system usually are concentrated in the lower part of the respiratory system and thus difficult to reach for taking a sample. This may also be the case for certain bacterial diseases. Accordingly, the number of pathogens to detect from a sample collected from mouth or nose of a patient is not sufficient as most of the sample derives from the wrong upper part of the respiratory system. Secondly, the time given to the patient for giving a sample is believed to be too short so that not sufficient pathogens can accumulate in the sample. Furthermore, at least the cotton swap method Is ineffective, as the major fraction of pathogens contained in a sample will most likely irreversibly stick to the cotton swab, which is made from cellulose, which is known to have high adsorbent potency, further decreasing the number of detectable pathogens in a sample. Further, the cotton swap will only gather a minuscule part of fluid, more or less in arbitrary regions of the mucosa, resembling a more random method.

Applicant now has surprisingly found that a completely different way of taking a sample will lead to a more reliable detection of infections of the respiratory tract. This involves taking a sample using a kind of respiratory mask which has been adapted to contain a means for collecting a sample according to the invention.

Conventional respiratory masks normally include a filter sheet that is permeable to air and that is exposed to the atmosphere so as to permit air flow therethrough upon breathing. Equipped with a coated insert according to the invention, such respiratory mask is well-suited to collect samples to detect an infection of the respiratory tract. In order to achieve this, the mask according to the invention is placed on a patient's face so that at least the mouth is covered. The patient is then left to breathe and/or cough through the mask. A major part of pathogen particles and/or droplets in the patient's breath is directed to the coated insert, preferably located in front of the orofacial openings, and will stick to the coating material which preferably is a sugar. After a time, the mask can be taken off, the insert be removed, e.g. using forceps, and placed into a tube for further detection according to well-known means, e.g. PCR or RT PCR or electron microscopy. The present invention has the advantage that pathogens such as viruses are immobilized and can accumulate on the film over time and is preferably well conserved in a matrix, which is soluble in water and, upon contact with an aqueous fluid, will release a greater fraction of pathogens as other commonly used materials such as cotton. For example, the patient can be left with the mask for a time such as 10 to 30 minutes, preferably longer so that a substantial fraction of viruses transported through the breath will be caught on the coated insert. Also, viruses contained in mucus coughed into the mask, in particular onto the coated insert, can be collected on the insert.

Furthermore, after removal of the film from the mask body, pathogen particles sticking on it may be removed from said insert for further detection simply by rinsing them off. The nature of the coating material will lead to said material at least partially dissolving, so that a near quantitative yield of viral particles can be achieved. The sugar will be treated together with any pathogen particles in the further detection. However, it will not disturb or distort the result. It is believed that the present means for collecting a sample will be efficient both for viral and for bacterial pathogens. Examples for viruses of the respiratory tract include SARS-1, MERS, influenza or the novel coronavirus SARS-CoV-2 (Covid19), Ebola, Lassa and other diseases caused by adenovirus, picornaviridae and/or paramyxoviridae, those for bacterial diseases include tuberculosis and meningitis, pneumococcus, haemophilus influenzae, Moraxella catarrhalis and pertussis.

All in all, with the face mask according to the invention, detection of viral or bacterial pathogens causing an infection of the respiratory tract is more reliable so that infections may be detected at a much earlier stage as compared to known methods which is saving money and time and enables for earlier treatment of patients with a positive result.

The insert may be made of any suitable material. Said material is preferably hydrophilic in order to facilitate coating of the film with a water-soluble material which is able to immobilized pathogen particles. Said immobilization is realized in that viral particles are directed towards said coated insert and will stick to the coating material.
In another embodiment, said material is hydrophobic. In such cases, the coating material, during the coating process, either comprises a surfactant or said hydrophobic material is hydrophilized prior to coating by means commonly known in the art so that a film-coating can be achieved.

In a preferred embodiment, said insert is a film which is preferably made of hydrophilic or hydrophilized plastic. Examples of suitable plastics include polyether, polyethylene terephthalate polypropylene, polycarbonate, polystyrene, polyvinyl alcohol, polyester, polyamide, poly vinyl pyrrolidone, polyurethane and polyvinylchloride. Other suitable materials for said insert include glass or metal which may equally be coated.

In a preferred embodiment, said insert is embodied as a film. Depending on the size of the face mask, said film can be of any suitable size. Generally, the film will have a size of between 2 and 30 cm².

In another preferred embodiment, said insert is embodied as a fibrous pellet, mesh or other kind of network. In one alternative of this embodiment, said fibrous pellet, mesh or other kind of network may be located in a housing which itself is attached to the inside said mask body. Such housing will be embodied such that the insert is exposed in the direction to the patient's mouth so that the patient's breath or cough will contact said insert. In another alternative of this embodiment, said fibrous pellet, mesh or other kind of network is directly attached to the mask body at the side facing the patient's mouth.

In any case, said insert is preferably not in contact with the patient, in particular not the patient's mouth. However, short time contacts are unavoidable, in particular during placing the mask in the face, and are acceptable.

The coating material can be any water-soluble material which, when present in an aqueous solution, can be dried to form coatings. It may be advantageous to use a liquid with a higher vapor pressure than water such like alcohol or mixtures thereof with water. Preferably, the coating material can reversibly absorb water or aqueous solutions.

Such material is coated onto the film using methods well-known in the art. For example, an aqueous solution comprising said coating material is spread onto said film and then dried so that a coating is formed. Examples of suitable coating materials include sugars and amino acids or mixtures thereof.

In a preferred embodiment said coating material is a sugar. In this embodiment, viral or bacterial particles in their humid environment (breath or mucus) arrive at the coated insert where the coating sugar is partially dissolved. This leads to the particles being incorporated into a sugar matrix, whereas excess humidity will evaporate again. As a side effect, this also leads to the virus or bacterial particles being protected from drying out and from other environmental factors leading to their destruction. Pathogen particles are thus conserved until detection is carried out.

The sugar may be any sugar the presence of which is compatible with the chosen detection method. Sugars include glucose, saccharose, trehalose, galactose, mannose and dextrose. Preferably, the sugar is selected from the group consisting of glucose, saccharose and trehalose, most preferably saccharose or glucose. In the preparation of the coating material comprising glucose, glucose syrup may advantageously be used.

It is preferred, that the coating material comprises at least 30 wt.-% of said sugar, preferably at least 40wt.-%, more preferably at least 50 wt.-%. For the avoidance of doubt, these percentage indicate the amount of sugar in dried form.

Depending on the physical properties of the sugar, it may be necessary to add at least one further ingredient to the coating material in order to prevent the coating from becoming brittle and chipping off the - possibly flexible - insert. Further properties which may be positively affected include stability and water-resorption properties. Suitable substances to be mixed into the coating material in order to influence these properties include polysaccharides such as carrageenan, arabinogalactan, dextran, gum guar, gum locust bean, gum karaya, inulin, mannan, pectin, starch, xanthan or xylan as well as gelatin, cellulose derivatives like hydroxypropyl cellulose, hydroxypropyl cellulose, hypromellose, polyethylene glycol, glycerol. These substances are useful because of their gelling, thickening, and stabilizing properties.

Further stabilizing agents may be added like magnesium or sodium thioglycolate. The matrix may further be stabilized by adding proteins like albumin or macromolecular constituents like carbomers as well as plant lectins.

The face mask may be disposable or re-usable. Both disposable and re-usable masks according to the invention comprise an insert which is detachable from the mask body. In case of a re-usable mask body, the mask body is preferably made from a material that can be sterilized for further use.

In a preferred embodiment, the face mask according to the invention can covers a patient's nose. In this embodiment, both particles coming out of a patient's mouth and nose are able to accumulate on the coated insert by immobilizing. In order to further improve accumulation for masks which cover only the patient's mouth, passage through the nose may be blocked for the time during which a sample is taken. This is not necessary and thus more comfortable for the patient in cases where the face mask covers both mouth and nose.

Whereas it is possible that the mask is held attached to the patient's face by pressing it against said face, it is preferable that said mask body has at least one means for attaching the face mask to a patient's face. In a more preferred embodiment, such at least one means for attaching is at least one strap hole at the opposite sides of said mask body. The ends of a strap may be fastened using at least one hole at each side of said mask body so that the mask can be fixed to the patient's face. In some embodiments, each side of the mask body may comprise two strap holes, preferably an upper and a lower strap hole which will lead to a more comfortable attachment of the face mask to the face. Usually the mask is fixed by straps around the head or behind the ears. In another more preferred embodiment, such at least one means for attaching is a strap attached to said mask body. In such an embodiment, said means may be an extension of said mask body.

The insert is, preferably reversibly, attached to said mask body. In a preferred embodiment, said insert, in particular in case it is embodied as a film, is at least partially covering said mask body. This means that a substantial part of the area of said insert or film is attached to said mask body. In any case attachment is such that the insert can be removed from the mask body for further detection. Attachment may be effected e.g. by an adhesive, velcro or a hook-and-loop fastener.

In a preferred embodiment, the material of said mask body is air-permeable. In this way, evaporation of excess humidity from the patient's breath is improved.

The mask body according to the invention may be flexible or have a pre-defined three-dimensional structure. In both cases, the mask body may be foldable which is especially advantageous in order to safe space, e.g. during transportation.

Thus, in one preferred embodiment, said mask body is flexible. Suitable materials for the mask body include cellulose or plastic fibers, open porous foam materials or elastomers, optionally combined with filters.
In another preferred embodiment, said mask body has a predefined three-dimensional shape. An exemplary and preferred pre-defined three-dimensional shape is such that the mask body is shaped such that the patient's breath is guided towards the coating material. Preferably an inner width of said mask body is reducing towards the side opposite to the patient's mouth. More preferably, the area opposite the patient's mouth is also shaped such that the insert is located opposite the patient's mouth, either as a flat or curved film, both along the shape of the mask body, or as a fibrous pellet, mesh or other network. Such mask bodies having a pre-defined shape may be made from a thermoplast, other plastic or silicone.

In another embodiment, the present invention relates to a method for collecting a sample from the respiratory tract of a patient comprising providing a face mask according to the present invention, attaching it to a patient's face and collecting the sample.

In yet another embodiment, the present invention relates to the use of the face mask according to the invention for collecting a sample from the respiratory tract of a patient. Such sample may be further used in detection methods for viral or bacterial pathogens of the respiratory tract as known in the art.

In another aspect, the present invention relates to an insert attachable to a mask body as described in any one of claims 1, 9 to 11 and 13 to 17 or a face mask or respiratory mask, wherein said insert is attached to said mask body or mask at the side facing a patient's mouth and wherein said insert is covered with a material able to accumulate pathogen particles. Said insert is embodied as described in connection with the face mask according to the invention of which such insert is an integral part.

The present invention also relates to the use of the insert according to the invention for taking a sample from the respiratory tract of a patient in connection with a face mask or respiratory mask.

In connection with this, the present invention relates to a kit-of-parts comprising a mask body as described herein or a face mask or respiratory mask and at least one insert as described herein. Optionally, said kit further comprises a suitable container for hosting said mask and at least one insert.

Illustrative embodiments according to the invention are shown in the figures and explained in greater detail below.

### Brief description of the drawings

Figure 1 shows a first face mask according to the invention in longitudinal section.
Figure 2 shows a view onto the inner surface of the mask of Fig. 1.
Figure 3 shows a second face mask according to the invention in longitudinal section.
Figure 4 shows a view onto the inner surface of the mask of Figure 3.
Figure 5 shows a third face mask according to the invention in front view.
Figure 6 shows a fourth face mask according to the invention with a view towards the inner side.
Figure 7 shows a fifth face mask according to the invention having an alternative insert with a view onto the inner surface.

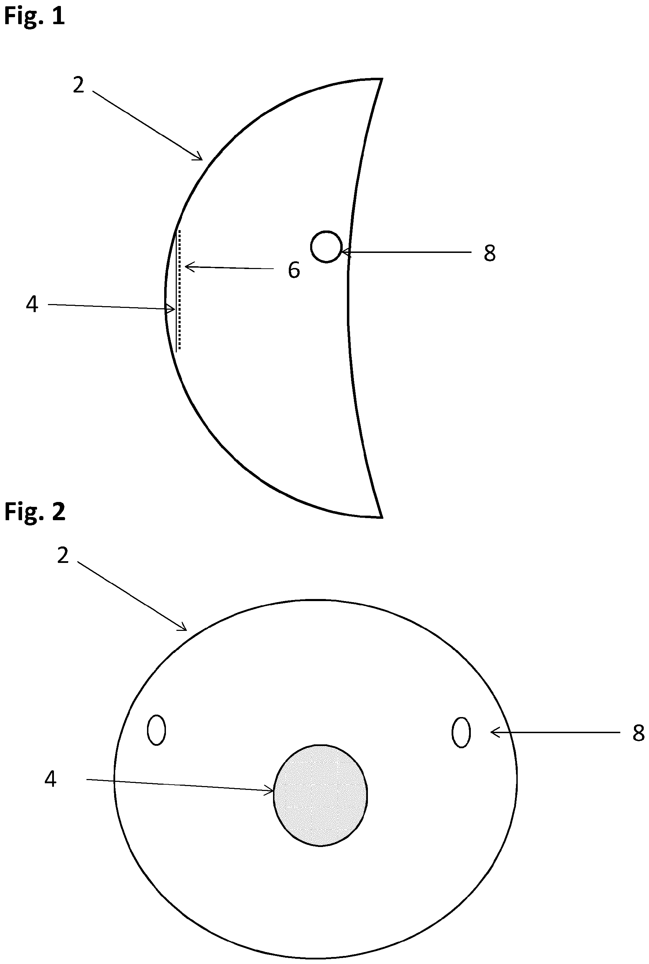

Figure 1 shows a first embodiment of a face mask according to the invention in longitudinal section. The mask body 2 is shaped as a hemisphere with a hollow internal space. The mask body is flexible or pre-defined and can be made of any material able to provide one of these properties. In this embodiment, the insert 4 is embodied as a film which is attached directly to the inner wall of the mask body. The exact location is such that said film 4 is directed at the patient's mouth with its surface. Said film 4 may be quadrangular, round, triangular or have any two-dimensional shape which fits into the mask body 2. Generally, the film 4 may be attached to the mask body 2 at one or more places, in the current case, it is attached at two spaces, or even with its complete surface. In the latter case, the film, in its three-dimensional arrangement will adapt to the shape of the mask body. The film 4 is coated with a material able to accumulate pathogen particles or body cells 6. Such coating 6 is present at least on the surface of the film directed towards the patient's mouth. Typically, a coating is between 0.1 and 4 mm thick, preferably between 0.2 and 2 mm. The thickness largely depends on the coating material. Thicker coating can usually be obtained with more viscous starting materials or by repeating coating steps. At the lateral side the embodiment of Figure 1 is embodied with one strap hole 8 which is suitable to attach at least one fastening means such as a strap to the mask body 2. In other embodiments, two strap holes may be more suitable to attach the face mask to the patient's face.

Figure 2 displays a view towards the inner surface of the face mask according to figure 1. The insert 4 is placed in the middle of the mask body 2 where it can be attached at single points or edges or with a greater portion of its surface. The latter may be in particular the case where the insert 4 is made of flexible material or formed according to the shape of the mask body to fit into a specific position of the mask body. At both lateral sides, the mask body is equipped with one strap hole 8 to host any kind of fixing device suitable to attach the face mask to a patient's head.

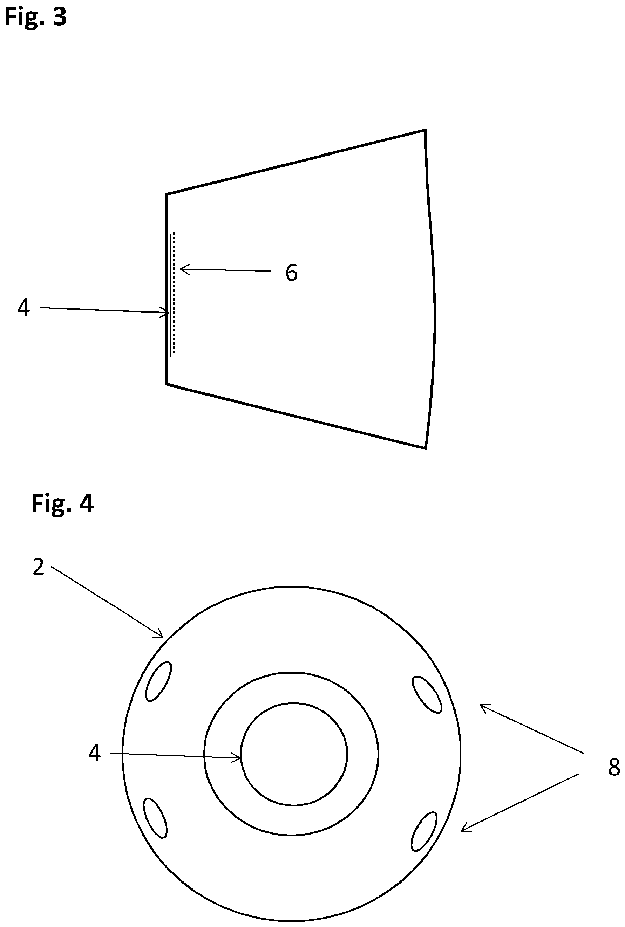

Figure 3 shows a second face mask according to the invention in longitudinal section. The mask body 2 is shaped as a four-sided distorted frustrum with an opening to host at least the patient's mouth at the quadrangle with the widest diameter, or as a truncated cone with the opening to host at least the patient's mouth at the circle with the widest diameter. The insert 4 with the coating material 6 showing towards the hollow space of the face mask is adapted to fit onto the side having the lowest diameter which is located opposite of the patient's mouth and optionally nose.

Fig. 4 displays a view towards the inner surface of the face mask according to figure 3. The insert 4 is placed in the middle of the surface of the mask body 2 located opposite to the patient's mouth and optionally nose. At both lateral sides of this embodiment, the mask body 2 is equipped with a pair of strap holes 8 to host any kind of fixing device suitable to attach the face mask to a patient's head.

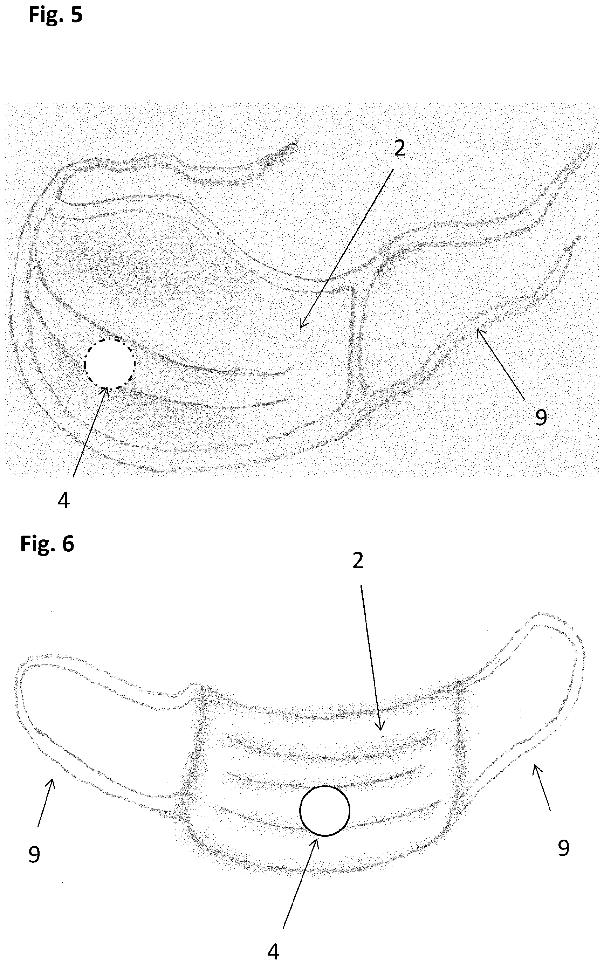

Figure 5 shows a third face mask with a view onto the outer surface. This face mask is embodied with a mask body 2 made of flexible material such as cellulose or a cellulose derivative which is shaped with pleats to allow for extension of the inner hollow space of the face mask. Instead of strap holes and optionally straps attached to said mask body in order to attach it to a patient's mouth and optionally nose, this face mask is embodied with a pair of straps 9 at each lateral side which form a part of the mask body 2. The insert 4 (dashed line) is hidden inside of the mask body 2.

Figure 6 shows a further face mask according to the invention with a view to the inner side of said mask. The mask is embodied with a mask body 2 made of flexible material shaped with pleats to allow for extension of the inner hollow space of the face mask. The insert 4 is placed onto the mask body 2 in order to ensure optimal sample collection from the patient's mouth and optionally nose. In contrast to the face mask according to figure 5, the pair of straps 9 on each lateral side pass into each other to form a loop which can be wound around a patient's ear for attachment.

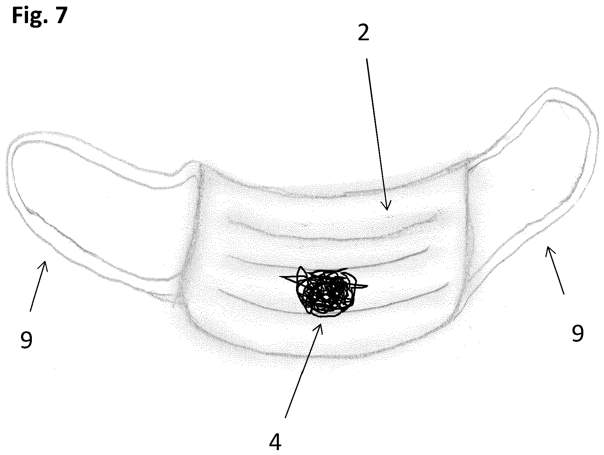

Figure 7 shows another face mask according to the invention with a view to the inner side of said mask. The mask is embodied with a mask body 2 made of flexible material shaped with pleats to allow for extension of the inner hollow space of the face mask. The insert 4 is shaped as a fibrous pellet which is coated with appropriate coating material. The insert 4 attached onto the inner side of the mask body 2 in order to ensure optimal sample collection from the patient's mouth and optionally nose.

The pair of straps 9 on each lateral side pass into each other to form a loop which can be wound around a patient's ear for attachment.

## Claims

1. Face mask for collecting a sample from a patient comprising
- a mask body comprising at least one outer layer able to cover at least mouth of a patient's face,
- an insert attached to said mask body at the side facing the patient's mouth and covered with a material able to accumulate pathogen particles.

2. Face mask according to claim 1, wherein said insert comprises a film, a mesh, a fibrous pellet or other network.

3. Face mask according to claim 1 or 2, wherein said insert is made of plastic, preferably hydrophilic or hydrophilized.

4. Face mask according to claim 3, wherein said plastic is selected from the group consisting of polyether, polyester, polyamide, polyethylenterephthalate polypropylene, polycarbonate, polystyrole, polyvinyl alcohol, polyvinyl pyrrolidone, polyurethane and polyvinylchloride.

5. Face mask according to any one of claims 1 to 3, wherein said material is a sugar.

6. Face mask according to claim 5, wherein said sugar is selected from saccharose, glucose, trehalose, galactose mannose and dextrose.

7. Face mask according to any of the preceding claims, wherein said insert is detachable.

8. Face mask according to any of the preceding claims, wherein said face mask is able to cover a patient's nose.

9. Face mask according to any of the preceding claims, wherein said mask body has at least one means for attaching the face mask to a patient's face.

10. Face mask according to claim 9, wherein said at least one means for attaching is at least one strap hole at the opposite sides of said mask body.

11. Face mask according to claim 10, wherein each side comprises a pair of upper and lower strap holes.

12. Face mask according to any of the preceding claims, wherein said insert is at least partially covering said outer layer.

13. Face mask according to any of the preceding claims, wherein the material of said mask body is air permeable.

14. Face mask according to any of the preceding claims, wherein said mask body is flexible.

15. Face mask according to claim 14, wherein said mask body is made of cellulose or plastic fibers, open porous foam materials or elastomers.

16. Face mask according to any of the preceding claims wherein said mask body has a predefined three-dimensional shape.

17. Face mask according to claim 16, wherein said three-dimensional shape is such that the mask body is reducing towards the side opposite to the patient's mouth.

18. Method for collecting a sample from the respiratory tract of a patient comprising providing a face mask according to any of the preceding claims, attaching it to a patient's face and collecting the sample.

19. Use of the face mask of any one of claims 1 to 16 for collecting a sample from the respiratory tract of a patient.

20. An insert attachable to a mask body as described in any one of claims 1, 9 to 11 and 13 to 17 or a face mask or respiratory mask, wherein said insert is covered with a material able to accumulate pathogen particles.

21. Kit-of-parts comprising a mask body according to any one of claims 1, 8 to 11, 13 to 17 or a face mask or respiratory mask and at least one insert according to 2 to 7 and 12.
